# EUROPEAN PATENT APPLICATION

(11) **EP 4 732 910 A1**
(43) Date of publication of application: **29.04.2026**
(21) Application number: 24209082.7
(22) Date of filing: 26.10.2024
(51) Int. Cl.: A61Q 19/00, A61K 8/06, A61K 8/14, A61K 8/49, A61K 8/67, A61K 8/73

(54) **COSMETIC COMPOSITION CONTAINING VITAMIN B12 ENCLOSED IN LIPOSOMES**

(71) Applicant: Alkemie Group 1 Spolka z o.o., 81-512 Gdynia (PL)
(72) Inventor: ZUKOWSKA-BODNAR, Justyna, 58-500 Jelenia Góra (PL)
(74) Representative: Czub, Krzysztof

(57) **Abstract**

The invention is related to a cosmetic composition containing vitamin B12 enclosed in liposomes. The invention relates to the field of cosmetology and is referred to the technology for obtaining cosmetic skin care products with moisturizing properties. It is particularly desirable in the care of problematic skin, prone to irritation, affected by rosacea, atopic or eczema. The cosmetic composition containing vitamin B12, is characterized that include from 3 to 5 phases, said composition comprising: from 60% to 90%, by weight, of phase A which is a hydrophilic phase; from 5% to 30%, by weight, of phase B which is a hydrophobic phase; from 0.5% to 1%, by weight, of phase C and D have containing moisturizing ingredients, and from 4.5% to 5.5%, by weight, of last phase (C or E) with vitamin B12 which is enclosed in liposomes. The vitamin B12 favourably is cyanocobalamin.

## Description

### FIELD OF THE INVENTION

The subject matter of the invention is a cosmetic composition containing vitamin B12 enclosed in liposomes. The invention relates to the field of cosmetology and is referred to the technology for obtaining cosmetic skin care products with moisturizing properties. It is particularly desirable in the care of problematic skin, prone to irritation, affected by rosacea, atopic or eczema.

### BACKGROUND OF THE INVENTION

Vitamin B12 belongs to a group of chemical compounds that contain a corin system (four pyrrole rings with a cobalt atom in the center). Its synthesis is possible only in prokaryotic cells (bacteria and archaea), but it is necessary for the proper functioning of the human body. The source of vitamin B12 are animal products and partially fermented vegetables (fermented soybeans) or some algae. The daily requirement of an adult for cyanocobalamin is on average 2 mg (varies between countries). Vitamin B12 is stored in tissues, mainly in the liver, and its deficiencies can occur 5 years after taking the last dose. Deficiencies are primarily faced by vegans and vegetarians and people with absorption disorders in the digestive tract.

Absorbed in the final section of the intestine, cobalamin plays a huge role in the proper functioning of the body. By combining with glycoprotein, it creates a hematopoietic factor, participating in haematopoiesis (the process of blood cell formation in the hematopoietic system) and erythropoiesis (the process of red blood cell formation in the bone marrow). In the nervous system, it participates in the construction of the myelin sheath and the synthesis of neurotransmitters. It takes part in the metabolic transformations of carbohydrates and fats. Additionally, it takes part in the synthesis of deoxyribonucleic acid and ribonucleic acid in erythroblasts and in the metabolic processes of DNA (metabolism of pyrimidines and purines). Vitamin B12 deficiency results in a greater possibility of DNA damage, increases the level of homocysteine, which is a significant risk factor in circulatory system diseases. Other physiological symptoms of cobalamin deficiency include impaired haematopoiesis, possible severe neurological disorders with limb paralysis, polyneuritis, ataxia and lethargy.
In addition to its effects on the circulatory and nervous systems, vitamin B12 affects the condition of the skin and the processes occurring in it: supports wound healing - thanks to its participation in DNA synthesis, it plays a significant role in tissue regeneration processes; reduces inflammation - cyanocobalamin has anti-inflammatory properties that can help alleviate skin inflammation, such as acne, atopic dermatitis or eczema; reduces the risk of hyperpigmentation and evens out skin tone; strengthens the skin barrier, helps maintain an appropriate level of moisture and protection against the harmful effects of external factors and helps maintain skin elasticity, thus slowing down the visible signs of skin ageing.

Vitamin B12 seems to be particularly helpful in the care of skin prone to rosacea or atopic dermatitis. Such skin is characterized by weakened cell defense mechanisms against external environmental factors and free radicals. As a result, the barrier function of the skin deteriorates, and consequently, disease symptoms such as exacerbated inflammatory reactions, dryness, skin flaking, and skin dermatoses intensify. Randomized studies conducted on a group of probands affected by eczema confirmed that local application of preparations with 0.07% vitamin B12 resulted in a significant reduction in erythema, itching, and skin dryness compared to probands using placebo.
In vitro studies indicate that cyanocobalamin may have an inhibitory effect on the production of cytokines by T lymphocytes, which are responsible for inducing inflammatory processes accompanying eczema.

The available studies do not provide precise information on the penetration of cyanocobalamin into the epidermis but analysing the structure and chemical properties of the substance (highly hydrophilic nature, high molecular weight) it can be assumed that it is low.
In addition, studies were conducted on the effect of vitamin B12 in toothpastes. Randomized studies were conducted on a group of 103 people whose task was to use toothpaste containing cobalamin. After the experiment, the level of vitamin B12 in serum was determined. The analysis showed that in the group that did not receive placebo, the level of cyanocobalamin increased by an average of 23%, which was an additional daily dose of approximately + 7 µg of oral vitamin B12. It can be assumed with great certainty that the route of administration is responsible for this state of affairs; as is known, the oral mucosa is much more permeable to chemicals than the stratum corneum of the epidermis.

There are known solutions of cosmetic compositions containing, among others, vitamin B12 about the beneficial effects on the skin. It is particularly desirable in the care of problematic skin, prone to irritation, affected by rosacea, atopic or eczema.

The description of the invention application PL444345A1 discloses a cosmetic composition for external use containing a combination of dry extract of Asian pennywort and vitamin B12, and its use in cosmetic products. The vitamin B12 contained in them soothes sensitive and irritated skin, reducing existing inflammatory reactions.

According to patent CN101669888A it is known a cosmetics containing vitamin B12 and vitamin E. Vitamin B12 in theses cosmetics provides a good effect for dispelling wrinkles and/or scars.

The cosmetics products with vitamin B12 are available on the cosmetic market, but no company offers this form of this vitamin enclosed in liposomes.

### SUMMARY OF THE INVENTION

The object of the present invention is to provide cosmetic composition containing vitamin B12 encapsulated in liposomes with moisturizing properties and is characterized that with better absorption of vitamin B12 in the deeper layers of the epidermis and ensuring strengthening of the hydrolipid layer of the epidermis.

### DETAILED DESCRIPTION OF THE INVENTION

The invention essence lies in the fact that in a cosmetic composition containing vitamin B12 is characterized that from 3 to 5 phases, said composition comprising:
a) from 60% to 90%, by weight, of phase A which is a hydrophilic phase;
b) from 5% to 30%, by weight, of phase B which is a hydrophobic phase;
c) from 0.5% to 1%, by weight, of phase C and D have containing moisturizing ingredients;
d) from 4.5% to 5.5%, by weight, of last phase (C or E) with vitamin B12 which is enclosed in liposomes.

The vitamin B12 favourably is cyanocobalamin.

The vitamin B12 favourably is enclosed in liposomes which pre-liposomes include aqua (water), lecithin, glycerine, pentylene glycol, sodium hydroxide and tocopherol.

The phase A or the phase C or the phase D favourably has also additional active ingredients with a moisturizing effect.

Preferable trehalose and sodium salt of low and high molecular weight hyaluronic acid, or PCA salts (allantoin and betaine or ectoine and ceramides) are included in four-phase or five-phase of cosmetic composition.

### ADVANTAGEOUS EFFECTS OF INVENTION

The advantage of the present invention is use of vitamin B12, which was previously enclosed in pre-liposomes. This procedure aims to facilitate the diffusion of the molecule in the epidermis, increase its stability and resistance to external factors such as temperature or the adverse effects of light. An additional advantage of this solution is the strengthening of the hydrolipid layer of the epidermis by embedding the phospholipid shell of the liposome in it. Vitamin B12 itself is released in the deeper parts of the epidermis gradually, the supply is extended over time, and thus the effect of the product after application is extended.

To prepare vitamin B12 enclosed in lipid spheres, ready-made pre-liposomes were used. This raw material meets all the safety requirements for chemical substances and mixtures in the European Union, is accepted in China, does not contain ingredients of animal origin, so it can also be used by vegans and vegetarians.

All proposed recipe examples are developed with ingredients with the Vegan declaration, so they can also be used by vegetarians and vegans - a group particularly vulnerable to vitamin B12 deficiency.

None of the proposed recipes contain a fragrance composition, because in our opinion it is a potential irritant.

The preservative system used in the sample recipes is not classified in accordance with Regulation (EC) No 1223/2009 of the European Parliament and of the Council of 30 November 2009 on cosmetic products as a preservative, it has an antioxidant effect. The recipes in Examples 2 to 5 meet the requirements of ISO 16128-1:2016 regarding the criteria for ingredients of natural origin.

### EXAMPLES

Example of method for the preparation of vitamin B12 encapsulated in liposomes

The procedure of encapsulating vitamin B12 in liposomes involves preparing an aqueous solution of cyanocobalamin.

For this purpose, a 0.100000% solution was used, then Natipide Eco was added to the solution in a ratio of 1:8 (w:w), thus obtaining liposomes containing 0.012500% of vitamin B12 in pure form.

To prepare vitamin B12 enclosed in lipid spheres, ready-made Natipide Eco preliposomes from Lipoid Kosmetik were used. Natipide Eco pre-liposomes contain aqua (water), lecithin, glycerin, pentylene glycol, sodium hydroxide, tocopherol. This raw material meets all the safety requirements for chemical substances and mixtures in the European Union, is accepted in China, does not contain ingredients of animal origin, so it can also be used by vegans and vegetarians.

With the assumed concentration of liposomes in the final product recipe at 5%, the concentration of vitamin B12 in pure form in cosmetic compositions is 0.000625%.

### Examples of cosmetics composition

The formulas from examples 2 to 5 meet the requirements of ISO 16128-1:2016 regarding the criteria for ingredients of natural origin.

Example 1 is a basic emulsion containing vitamin B12 encapsulated in liposomes.

The following examples additionally contain active ingredients with a moisturizing effect: example 3 (trehalose, sodium salt of low and high molecular weight hyaluronic acid) and example 5 - PCA salts, conditioning (example 2 - allantoin and betaine; example 4 - ectoine and ceramides).

### Example 1

Phase A components were introduced into the main tank, thoroughly mixed and heated to 75°C. Phase B components were introduced into the melter, thoroughly mixed while heating to 75°C. After reaching the target temperatures, phase B was transferred to phase A using a pump, and the whole was homogenized while mixing. After homogenization, the emulsion was cooled to 25°C. Before adding phase C to the solution, the components were thoroughly dispersed. Phase C was added while gently mixing. After checking the physicochemical parameters, the mass was poured into storage tanks, and the sample taken was sent for microbiological testing. The mass is released for packaging after obtaining the results of all tests. The quantitative and qualitative composition of the phases is presented in Table 1.

**Table 1. Quantitative and qualitative (% w/w) composition of the first composition.**

| **Phase** | **Ingredients** | **INCI Names** | **Amount (weight percent)** |
|---|---|---|---|
| **A** | Production water | Aqua | 76.850000 |
| | Dermofeel PA3 | Sodium Phytate | 0.050000 |
| | A-Leen 5 | Pentylene Glycol | 3.000000 |
| | Vegetable glycerin (99.5%) | Glycerin | 3.000000 |
| | Symsave H | Hydroxyacetophenone | 1.000000 |
| | Sepinov EMT 10 | Hydroxyethyl Acrylate/Sodium Acryloyldimethyl Taurate Copolymer | 0.800000 |
| | Sepimax Zen | Polyacrylate Crosspolymer-6 | 0.300000 |
| **B** | Crodamol ISIS-LQ | Isostearyl Isostearate | 5.000000 |
| | LexFeel^{™} N5 MB | Diheptyl Succinate (and) Capryloyl Glycerin/Sebacic Acid Copolymer | 5.000000 |
| **C** | Natipide Eco | Aqua (water), Lecithin, Glycerin, Pentylene Glycol, Sodium Hydroxide, Tocopherol | 4.375000 |
| | CetPro VB12 | Vitamin B12 | 0.000676 |
| | Production water | Aqua | 0.624320 |

### Example 2

Phase A components were introduced into the main tank, thoroughly mixed and heated to 75°C. Phase B components were introduced into the melter, thoroughly mixed while heating to 75°C. After obtaining the desired temperatures, phase B was transferred to phase A using a pump and the whole was homogenized while stirring. After homogenization, the emulsion was cooled to 60°C and the phase C components were added. The whole was cooled to 25°C, the phase D component and the previously prepared phase E components were added. Before adding phase E to the solution, the components were thoroughly dispersed. After checking the physicochemical parameters, the mass was poured into storage tanks and the sample taken was sent for microbiological testing. The mass is released for packaging after obtaining the results of all tests. The quantitative and qualitative composition of the phases is presented in Table 2.

**Table 2. Quantitative and qualitative (% w/w) composition of the second composition.**

| **Phase** | **Ingredients** | **INCI Names** | **Amount (weight percent)** |
|---|---|---|---|
| **A** | Production water | Aqua | 59.950000 |
| | Dermofeel PA3 | Sodium Phytate | 0.100000 |
| | A-Leen 5 | Pentylene Glycol | 1.000000 |
| | Vegetable glycerin (99.5%) | Glycerin | 4.000000 |
| | RonaCare Allantoin | Allantoin | 0.050000 |
| | Genencare OSMS BH | Betaine | 0.500000 |
| | Keltrol CG-V | Xanthan Gum | 0.300000 |
| | Niacinamide | Niacinamide | 5.000000 |
| **B** | Crodamol ISIS-LQ | Isostearyl Isostearate | 5.000000 |
| | Crodamol GTCC | Caprylic/Capric Triglyceride | 5.000000 |
| | Tocopherol acetate | Tocopheryl Acetate | 0.100000 |
| | Floraesters K-100 Jojoba | Hydrolyzed Jojoba Esters, Jojoba Esters, Water | 0.500000 |
| | Montanov 202 | Arachidyl Alcohol, Behenyl Alcohol, Arachidyl Glucoside | 4.500000 |
| | Tegin 4100 Pellets | Glyceryl Stearate | 1.500000 |
| | Lanette O | Cetearyl Alcohol | 2.500000 |
| | Neossance Squalane | Squalane | 1.000000 |
| | Oilnat Grapeseed Premium Refined | Vitis Vinifera Seed Oil | 2.000000 |
| **C** | Symsave H | Hydroxyacetophenone | 1.000000 |
| **D** | D-Panthenol (75%) | Panthenol | 1.000000 |
| **E** | Natipide Eco | Aqua (water), Lecithin, Glycerin, Pentylene Glycol, Sodium Hydroxide, Tocopherol | 4.375000 |
| | CetPro VB12 | Vitamin B12 | 0.000676 |
| | Production water | Aqua | 0.624320 |

### Example 3

Phase A components were introduced into the main tank, thoroughly mixed and heated to 75°C. Phase B components were introduced into the melter, thoroughly mixed while heating to 75°C. After obtaining the desired temperatures, phase B was transferred to phase A using a pump and the whole was homogenized while stirring. After homogenization, the emulsion was cooled to 60°C and the phase C components were added. The whole was cooled to 25°C, the phase D component and the previously prepared phase E components were added. Before adding phase E to the solution, the components were thoroughly dispersed. After checking the physicochemical parameters, the mass was poured into storage tanks and the sample taken was sent for microbiological testing. The mass is released for packaging after obtaining the results of all tests. The quantitative and qualitative composition of the phases is presented in Table 3.

**Table 3. Quantitative and qualitative (% w/w) composition of the third composition.**

| **Phase** | **Ingredients** | **INCI Names** | **Amount (weight percent)** |
|---|---|---|---|
| **A** | Production water | Aqua | 75.430000 |
| | A-Leen 5 | Pentylene Glycol | 3.000000 |
| | Vegetable glycerin (99.5%) | Glycerin | 3.000000 |
| | RonaCare Allantoin | Allantoin | 0.100000 |
| | Genencare OSMS BH | Betaine | 2.000000 |
| | Keltrol CG-V | Xanthan Gum | 0.250000 |
| | Trehalose 100 | Trehalose | 2.000000 |
| **B** | Polyaquol 2W | Polyglyceryl-2 Stearate, Glyceryl Stearate, Stearyl Alcohol | 5.000000 |
| | Lanette O | Cetearyl Alcohol | 1.500000 |
| | Radiacid 0417 | Stearic Acid | 1.200000 |
| **C** | Symsave H | Hydroxyacetophenone | 1.000000 |
| **D** | Production water | Aqua | 0.500000 |
| | Sodium Hyaluronate Low Molecular Weight | Sodium Hyaluronate | 0.010000 |
| | Sodium Hyaluronate High Molecular Weight | Sodium Hyaluronate | 0.010000 |
| **E** | Natipide Eco | Aqua (water), Lecithin, Glycerin, Pentylene Glycol, Sodium Hydroxide, Tocopherol | 4.375000 |
| | CetPro VB12 | Cyanocobalamin | 0.000676 |
| | Production water | Aqua | 0.624320 |

### Example 4

Phase A components were introduced into the main tank, thoroughly mixed and heated to 75°C. Phase B components were introduced into the melter, thoroughly mixed while heating to 75°C. After obtaining the desired temperatures, phase B was transferred to phase A using a pump and the whole was homogenized while stirring. After homogenization, the emulsion was cooled to 60°C and the phase C components were added. The whole was cooled to 25°C, the phase D component and the previously prepared phase E components were added. Before adding phase E to the solution, the components were thoroughly dispersed. After checking the physicochemical parameters, the mass was poured into storage tanks and the sample taken was sent for microbiological testing. The mass is released for packaging after obtaining the results of all tests. The quantitative and qualitative composition of the phases is presented in Table 4.

**Table 4. Quantitative and qualitative (% w/w) composition of the fourth composition.**

| **Phase** | **Ingredients** | **INCI Names** | **Amount (weight percent)** |
|---|---|---|---|
| **A** | Production water | Aqua | 54.850000 |
| | Dermofeel PA3 | Sodium Phytate | 0.100000 |
| | A-Leen 5 | Pentylene Glycol | 3.000000 |
| | Vegetable glycerin (99.5%) | Glycerin | 3.000000 |
| | RonaCare Allantoin | Allantoin | 0.050000 |
| | Keltrol CG-V | Xanthan Gum | 0.300000 |
| | Sensocel Stab 26 | Microcrystalline Cellulose, Xanthan Gum | 3.000000 |
| **B** | Emulium Kappa MB | Candelilla/Jojoba/Rice Bran Polyglyceryl-3 Esters, Glyceryl Stearate, Cetearyl Alcohol, Sodium Stearoyl Lactylate | 4.500000 |
| | Ginol 16 | Cetyl Alcohol | 2.000000 |
| | Neossance Squalane | Squalane | 1.000000 |
| | Cetiol CC | Dicaprylyl Carbonate | 20.000000 |
| | Octan tokoferolu | Tocopheryl Acetate | 0.100000 |
| | Floraesters K-100 Jojoba | Hydrolyzed Jojoba Esters, Jojoba Esters, Water | 0.500000 |
| **C** | Symsave H | Hydroxyacetophenone | 1.000000 |
| | Cerafluid | Triolein, Glyceryl Dioleate, Ceramide NP | 1.000000 |
| **D** | Production water | Aqua | 0.500000 |
| | Ectoin Cosmetic | Ectoin | 0.100000 |
| **E** | Natipide Eco | Aqua (water), Lecithin, Glycerin, Pentylene Glycol, Sodium Hydroxide, Tocopherol | 4.375000 |
| | CetPro VB12 | Cyanocobalamin | 0.000676 |
| | Production water | Aqua | 0.624320 |

### Example 5

Phase A components were introduced into the main tank, thoroughly mixed and heated to 75°C. Phase B components were introduced into the melter, thoroughly mixed while heating to 75°C. After obtaining the set temperatures, phase B was transferred to phase A using a pump and the whole was homogenized while mixing. The whole was cooled to 35°C, the phase C component and homogenized, then cooled to 25°C and the previously prepared phase D was added. Before adding phase D to the solution, the components were thoroughly dispersed. After checking the physicochemical parameters, the mass was poured into storage tanks and the sample taken was sent for microbiological tests. The mass is released for packaging after obtaining the results of all tests. The quantitative and qualitative composition of the phases is presented in Table 5.

**Table 5. Quantitative and qualitative (% w/w) composition of the fifth composition.**

| **Phase** | **Ingredients** | **INCI Names** | **Amount (weight percent)** |
|---|---|---|---|
| **A** | Production water | Aqua | 68.900000 |
| | Dermofeel PA3 | Sodium Phytate | 0.100000 |
| | Vegetable glycerin (99.5%) | Glycerin | 2.000000 |
| | Keltrol CG-V | Xanthan Gum | 0.300000 |
| | Ajidew NL-50 | Sodium PCA | 1.000000 |
| **B** | Tego Care PBS 6 | Polyglyceryl-6 Stearate, Polyglyceryl-6 Behenate | 3.000000 |
| | Tegosoft AC MB | Isoamyl Cocoate | 6.000000 |
| | Tegosoft DC MB | Decyl Cocoate | 3.000000 |
| | Abyssinian oil | Crambe Abyssinica Seed Oil | 5.000000 |
| | Tocopherol acetate | Tocopheryl Acetate | 0.200000 |
| | Floraesters K-100 Jojoba | Hydrolyzed Jojoba Esters, Jojoba Esters, Water | 0.500000 |
| **C** | A-Leen 5 | Pentylene glycol | 5.000000 |
| **D** | Natipide Eco | Aqua (water), Lecithin, Glycerin, Pentylene Glycol, Sodium Hydroxide, Tocopherol | 4.375000 |
| | CetPro VB12 | Cyanocobalamin | 0.000676 |
| | Production water | Aqua | 0.624320 |

## Claims

1. A cosmetic composition containing vitamin B12, **characterized in that** include from 3 to 5 phases, said composition comprising:
a) from 60% to 90%, by weight, of phase A which is a hydrophilic phase,
b) from 5% to 30%, by weight, of phase B which is a hydrophobic phase,
c) from 0.5% to 1%, by weight, of phase C and D have containing moisturizing ingredients,
d) from 4.5% to 5.5%, by weight, of last phase (C or E) with vitamin B12 which is enclosed in liposomes.

2. A composition according to claim 1, **characterized in that** the vitamin B12 is cyanocobalamin.

3. A composition according to claim 1 or 2, **characterized in that** the vitamin B12 is enclosed in liposomes which pre-liposomes include aqua (water), lecithin, glycerine, pentylene glycol, sodium hydroxide and tocopherol.

4. A composition according to claim 1 or 2 or 3, **characterized in that** the phase A or the phase C or the phase D has additional active ingredients with a moisturizing effect.

5. A composition according to claim 1 or 2 or 3 or 4, **characterized in that** in four-phase or five-phase of cosmetic composition, trehalose and sodium salt of low and high molecular weight hyaluronic acid, or PCA salts (allantoin and betaine or ectoine and ceramides) are included.
